(19) Europäisches Patentamt / European Patent Office / Office européen des brevets

(11) **EP 4 316 541 A1**

(12) **EUROPEAN PATENT APPLICATION**
published in accordance with Art. 153(4) EPC

(43) Date of publication:
**07.02.2024 Bulletin 2024/06**

(51) International Patent Classification (IPC):
**A61L 33/06** (2006.01)　　**A61L 31/10** (2006.01)
**A61L 33/14** (2006.01)　　**C08G 18/42** (2006.01)
**C08G 18/48** (2006.01)

(21) Application number: **22781148.6**

(52) Cooperative Patent Classification (CPC):
**A61L 31/10; A61L 33/0011; A61L 33/06;**
C08G 18/42; C08G 18/48

(22) Date of filing: **30.03.2022**

(86) International application number:
**PCT/JP2022/016184**

(87) International publication number:
**WO 2022/210938 (06.10.2022 Gazette 2022/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.03.2021　JP 2021061344
13.08.2021　JP 2021131859**

(71) Applicants:
• **Kyushu University, National University
Corporation
Nishi-ku
Fukuoka-shi
Fukuoka 819-0395 (JP)**
• **DKS Co. Ltd.
Kyoto-shi, Kyoto 600-8873 (JP)**

(72) Inventors:
• **TANAKA, Masaru
Fukuoka-shi, Fukuoka 819-0395 (JP)**
• **KOBAYASHI, Shingo
Fukuoka-shi, Fukuoka 819-0395 (JP)**
• **NISHIMURA, Ayao
Kyoto-shi, Kyoto 600-8873 (JP)**
• **SHIREN, Minari
Kyoto-shi, Kyoto 600-8873 (JP)**
• **NISHIURA, Masahito
Kyoto-shi, Kyoto 600-8873 (JP)**

(74) Representative: **Hoffmann Eitle
Patent- und Rechtsanwälte PartmbB
Arabellastraße 30
81925 München (DE)**

(54) **COMPOSITION FOR MEDICAL USE AND APPLICATION OF SAME**

(57) Provided is a medical composition capable of imparting biocompatibility to the surface of a medical instrument etc. and application of the medical composition. In an embodiment of the present invention, the medical composition contains a urethane resin, and the urethane resin has urethane bonds in the main chain and a polyoxyethylene structure in the main chain and/or side chains. In another embodiment of the present invention, the urethane resin has a structural unit derived from a polycarbonate polyol and a structural unit derived from an aliphatic isocyanate and also has at least one urea bond.

Fig. 1

EP 4 316 541 A1

**Description**

Technical Field

The present invention relates to a medical composition and use thereof.

Background Art

It is known that in general, when biological tissue, blood, or the like comes into contact with the surface of a material not derived from a living body, the material surface is recognized as a foreign substance, proteins in the biological tissue are non-specifically adsorbed to the surface and denatured, activation of the coagulation system, complement system, platelet system, and the like occurs, and adhesion of blood cells, such as platelets, onto the surface occurs. In recent years, focusing on the state of water molecules contained in the surface of a synthetic polymer having a specific structure, it has become clear that non-specific adsorption of a protein onto the surface and denaturation of the adsorbed protein can be prevented by forming a surface capable of containing water molecules in a state referred to as "intermediate water." It has become clear that this surface has a low frequency of adhesion of platelets or the like upon contact with blood or the like, and can, for example, inhibit inflammation that occurs upon contact with in vivo tissues (see, for example, Non-patent Literature (NPL) 1).

Since the presence of the intermediate water inhibits non-specific adsorption or the like of a protein to a material surface, biocompatibility (hemocompatibility) can be brought about. For example, various medical instruments are desired to be in a state of having the biocompatibility (hemocompatibility) described above in order to inhibit phenomena such as non-specific adsorption of proteins caused by contact of in vivo tissue, blood, etc. with the substrate surface of medical instruments or the like.

A general method for imparting biocompatibility to a surface of a medical instrument in contact with in vivo tissue, blood, or the like is, for example, a method comprising forming a substrate using a material having mechanical characteristics or the like suitable for constituting such a medical instrument and then applying a material having biocompatibility to the substrate surface by means of coating or the like to form a film thereon.

Citation List

Non-patent Literature

NPL 1: Journal of the Adhesion Society of Japan, Vol. 51 No. 9 (2015), pp.15-25

Summary of Invention

Technical Problem

A wide variety of medical instruments are required to have biocompatibility, and the materials that make up these medical instruments are equally diverse. Properties that need to be ensured simultaneously with biocompatibility vary from instrument to instrument, for example, depending on the type and application of the medical instruments. Thus, coating agents used in imparting biocompatibility to the surface of medical instruments are required to have various properties. In particular, there is an indefinite expectation for proposals for coating agents exhibiting biocompatibility with new properties.

However, the mechanism by which a substance exhibits biocompatibility is not necessarily clear, and the correlation between a specific structure of a substance and the development of biocompatibility is unclear. Thus, there are no guidelines for designing a coating agent that exhibits biocompatibility as described above, and whether a substance exhibits biocompatibility is clarified only by evaluating the actually synthesized substance.

From this viewpoint, there has been strong demand in the medical field for development of novel medical materials capable of imparting biocompatibility to the surface of various medical instruments.

The present invention was made in view of the current state of the art. An object is to provide a medical composition capable of imparting biocompatibility to the surface of medical instruments and application of the medical composition.

Solution to Problem

The present inventors conducted extensive research to achieve the object and found that the object can be achieved by applying a urethane resin with a specific structure. Then, they completed the present invention.

Specifically, the present invention includes, for example, the subject matter described in the following items.

Item 1.
A medical composition comprising a urethane resin, the urethane resin having a urethane bond in a main chain and a polyoxyethylene structure in the main chain and/or a side chain.
Item 2.
The medical composition according to Item 1, wherein the urethane resin has a structural unit derived from a polycarbonate polyol.
Item 3.
The medical composition according to Item 1, wherein the urethane resin has a structural unit derived from an isocyanate compound.
Item 4.
A medical composition comprising a urethane resin, the urethane resin having a structural unit derived from a polycarbonate polyol and a structural unit derived from an aliphatic isocyanate, and having at least one urea bond.
Item 5.
The medical composition according to Item 4, wherein the urethane resin has a polyoxyethylene structure in a main chain and/or a side chain.
Item 6.
The medical composition according to any one of Items 1 to 5, which is an aqueous dispersion.
Item 7.
A protein adsorption inhibitor comprising the medical composition of any one of Items 1 to 6.
Item 8.
A platelet adsorption inhibitor comprising the medical composition of any one of Items 1 to 6.
Item 9.
A medical fiber treatment agent comprising the medical composition of any one of Items 1 to 6.
Item 10.
A medical instrument comprising a film of the medical composition of any one of Items 1 to 6.
Item 11.
A medical fiber comprising the medical fiber treatment agent of Item 9.
Item 12.
A method for inhibiting protein adsorption, comprising forming a film of the medical composition of any one of Items 1 to 6 on a substrate.
Item 13.
A method for inhibiting platelet adsorption, comprising forming a film of the medical composition of any one of Items 1 to 6 on a substrate.
Item 14.
A method for treating a medical fiber, comprising treating a medical fiber with the medical composition of any one of Items 1 to 6.

Advantageous Effects of Invention

The medical composition according to the present invention imparts biocompatibility to the surface of medical instruments.

Brief Description of Drawings

Fig. 1 is a graph showing platelet adhesion properties of polymer compositions according to the present invention.
Fig. 2 is a graph showing the mechanical strength of polymer compositions according to the present invention.
Fig. 3 is a graph showing the weight increase rate of polymer compositions according to the present invention when the polymer compositions contain water.
Fig. 4 is a graph showing protein adhesion properties of polymer compositions according to the present invention.

Description of Embodiments

The following describes embodiments of the present invention in detail. In the present specification, the terms "comprising," "containing," and "having" include the concepts of comprising, containing, consisting essentially of, and consisting of.

## 1. Medical Composition

The medical composition according to the present invention contains a urethane resin and encompasses, for example, the following medical composition A and medical composition B. Medical Composition A: this composition contains a urethane resin, and the urethane resin has a urethane bond in the main chain and a polyoxyethylene structure in the main chain and/or side chain.
Medical Composition B: this composition contains a urethane resin, and the urethane resin has a structural unit derived from a polycarbonate diol and a structural unit derived from an aliphatic isocyanate and also has at least one urea bond.
The following describes embodiments of medical composition A and medical composition B.

Medical Composition A

Medical composition A contains a urethane resin. The urethane resin, as stated above, has urethane bonds in the main chain, and also has a polyoxyethylene structure either in the main chain or in side chains, or in both the main chain and side chains. This urethane resin is referred to below as "urethane resin A" contained in medical composition A.
Urethane resin A can be of any structure as long as urethane resin A has a polyoxyethylene structure. For example, urethane resin A may contain the same structural unit as that of a known urethane resin in its molecule.
Urethane resin A may have one, or two or more structural units derived from a polyol. Urethane resin A may also have one, or two or more structural units derived from an isocyanate compound. Urethane resin A may also have both one, or two or more structural units derived from a polyol and one, or two or more structural units derived from an isocyanate compound. A structural unit derived from a polyol can mean, for example, a group formed by removing the hydrogen atom from a hydroxyl group in a polyol. A structural unit derived from an isocyanate compound can mean, for example, a group formed by adding a hydrogen atom to the nitrogen atom in an isocyanate group in an isocyanate compound.
The polyol to form structural units derived from a polyol can be of any type. The polyol can be selected from, for example, a wide range of known polyols to form urethane resin. Examples of polyols include polycarbonate polyols, polyester polyols, polyether polyols, hydrocarbon polyols, and low-molecular-weight polyols with a molecular weight or 400 or lower.
The polycarbonate polyols are not particularly limited, and can be selected from, for example, a wide range of known polycarbonate polyols for forming urethane resins. Examples include polycarbonate polyols obtained by reacting a polyol compound with a carbonate compound. Examples of polyol compounds include aliphatic polyols and alicyclic polyols. Examples of carbonate compounds include carbonate derivatives, such as carbonate esters and phosgene.
The polycarbonate polyols are preferably those with a structure derived from an aliphatic polyol. Examples of aliphatic polyols include ethylene glycol, diethylene glycol, 1,4-butanediol, 1,3-butanediol, 2,3-butanediol, 1,3-propylene glycol, 1,2-propylene glycol, 1,6-hexanediol, 3-methyl-1,5-pentanediol, and neopentyl glycol.
The polycarbonate polyols may have one, or two or more structures derived from a polyol compound. The polycarbonate polyols may also have one, or two or more structures derived from a carbonate derivative.
Polyester polyols are not particularly limited and can be selected from, for example, a wide variety of known polyester polyols for forming urethane resins, such as polyester polyols that are esterified condensates made by reacting a polyol compound with a polycarboxylic acid and that are terminated with a hydroxyl group. Examples of polyol compounds for forming a polyester polyol include the same polyol compounds for forming a polycarbonate polyol. Examples of polycarboxylic acids include succinic acid, glutaric acid, adipic acid, sebacic acid, phthalic acid, isophthalic acid, terephthalic acid, trimellitic acid, tetrahydrofuran acid, endomethyltetrahydrofuran acid, and hexahydrophthalic acid.
Polyester polyols may have one, or two or more structures derived from a polyol compound. Polyester polyols may also have one, or two or more structures derived from a polycarboxylic acid.
Polyether polyols are not particular limited and can be selected from, for example, a wide variety of known polyether polyols for forming urethane resins, such as polyether polyols formed by performing addition polymerization of a polyol compound and an alkylene oxide. Examples of polyol compounds for forming a polyester polyol include the same polyol compounds for forming a polycarbonate polyol. Examples of alkylene oxides include those such as ethylene oxide, propylene oxide, and butylene oxide. If urethane resin A has a structural unit derived from a polyether polyol, the structural unit also corresponds to the polyoxyethylene structure in urethane resin A.
Polyether polyols may have one, or two or more structures derived from a polyol compound. Polyether polyols may also have one, or two or more structures derived from an alkylene oxide.
Hydrocarbon polyols are not particularly limited and can be selected from, for example, a wide variety of known hydrocarbon polyols for forming urethane resins. Examples of hydrocarbon polyols include those with their hydrocarbon chain terminated with a hydroxyl group, such as polybutadiene polyol, polyisoprene polyol, hydrogenated polybutadiene polyol, and hydrogenated polyisoprene polyol.

Examples of low-molecular-weight polyols with a molecular weight or 400 or lower include ethylene glycol, diethylene glycol, triethylene glycol, 1,2-propylene glycol, 1,3-propylene glycol, neopentyl glycol, 1,3-butanediol, 1,4-butanediol, 3-methylpentane diol (MPD), 1,6-hexanediol (1,6-HD), 1,8-octane diol, 2-methyl-1,3-propanediol, bisphenol A, hydrogenated bisphenol A, cyclohexane dimethanol, glycerin, and trimethylolpropane. If the structural unit derived from a polyol in urethane resin A is formed from a low-molecular-weight polyol with a molecular weight of 400 or lower, the content ratio of the polyoxyethylene structure in urethane resin A can be increased.

In urethane resin A, the polyol for forming the structural unit derived from a polyol preferably contains at least the polycarbonate polyol as described above. In this case, medical composition A can easily form a film with excellent water resistance and strength, and the film can easily inhibit non-specific adsorption of proteins and adsorption of platelets.

The content ratio of the structural units derived from a polycarbonate polyol (excluding the polyoxyethylene structure unit) in urethane resin A may be 80 mass% or higher, preferably 90 mass% or higher, more preferably 95 mass% or higher, and still more preferably 99 mass% or higher based on the total mass of the structural units derived from a polyol. The structural units derived from a polyol contained in urethane resin A may be only those derived from a polycarbonate polyol.

The polyol preferably has two or more hydroxyl groups, and particularly preferably two hydroxyl groups.

The isocyanate compound for forming a structural unit derived from an isocyanate compound can be of any type and can be selected from, for example, a wide variety of known isocyanate compounds for forming urethane resins. Examples of isocyanate compounds include aliphatic isocyanates, alicyclic isocyanates, and aromatic isocyanates.

The aliphatic isocyanates are not particularly limited and can be selected from, for example, a wide variety of known aliphatic isocyanates for forming urethane resins. Aliphatic isocyanates may be, for example, one, or two or more of the following: tetramethylene diisocyanate, dodecamethylene diisocyanate, hexamethylene diisocyanate (HMDI), 2,2,4-trimethylhexamethylene diisocyanate, 2,4,4-trimethylhexamethylene diisocyanate, lysine diisocyanate, 2-methylpentane-1,5-diisocyanate, and 3-methylpentane-1,5-diisocyanate.

The alicyclic isocyanates are not particularly limited and can be selected from, for example, a wide variety of known alicyclic isocyanates for forming urethane resins. The alicyclic isocyanates may be, for example, one, or two or more of the following: isophorone diisocyanate (IPDI), hydrogenated xylylene diisocyanate, 4,4'-dicyclohexylmethane diisocyanate (H12MDI), 1,4-cyclohexane diisocyanate, methyl cyclohexylene diisocyanate, and 1,3-bis(isocyanatemethyl) cyclohexane.

The aromatic isocyanates are not particularly limited and can be selected from, for example, a wide variety of known aromatic isocyanates for forming urethane resins. The aromatic isocyanates may be, for example, one, or two or more of the following: tolylene diisocyanate (TDI), 2,2'-diphenylmethane diisocyanate, 2,4'-diphenylmethane diisocyanate, 4,4'-diphenylmethane diisocyanate (MDI), 4,4'-dibenzyl diisocyanate, 1,5-naphthylene diisocyanate, xylylene diisocyanate, 1,3-phenylene diisocyanate, and 1,4-phenylene diisocyanate.

In urethane resin A, the isocyanate compound for forming a structural unit derived from an isocyanate compound is preferably an aliphatic isocyanate. In this case, medical composition A can easily form a film with excellent strength and can easily limit an increase in weight of the film when the film is immersed in an aqueous phase.

The content ratio of the structural unit derived from an aliphatic isocyanate in urethane resin A may be 80 mass% or higher, preferably 90 mass% or higher, more preferably 95 mass% or higher, and still more preferably 99 mass% or higher, based on the total mass of the structural units derived from an isocyanate compound. The structural units derived from an isocyanate compound contained in urethane resin A may be only those derived from an aliphatic isocyanate.

The isocyanate compound preferably has two or more isocyanate groups, and particularly preferably two isocyanate groups.

The isocyanate compound may have a polyoxyethylene moiety. In this case, urethane resin A can have a polyoxyethylene structure introduced.

As stated above, urethane resin A also has a polyoxyethylene structure at least at either the main chain or side chains, or at both the main chain and side chains. Specifically, urethane resin A has a polyoxyethylene unit either in the main chain or in side chains, or in both the main chain and side chains. The polyoxyethylene structure is present by being covalently bound to at least either the main or side chains, or both, of urethane resin A.

For urethane resin A, the number average molecular weight of the polyoxyethylene unit is not particularly limited and may be, for example, 100 to 10000, preferably 150 to 5000, and more preferably 200 to 3000. The number average molecular weight of the polyoxyethylene unit referred to in the present specification means a value as measured by GPC.

As described later, urethane resin A with a polyoxyethylene structure can be obtained, for example, by using polyethylene glycol (PEG) or branched polyethylene glycol (PEG) as a starting material during polymerization reaction of urethane resin. Alternatively, urethane resin A with a polyoxyethylene structure can be obtained by reacting urethane resin with polyethylene glycol (PEG) or branched polyethylene glycol (PEG).

In urethane resin A, the content ratio of the polyoxyethylene structure (i.e., the content ratio of the polyoxyethylene unit) is not particularly limited. The content ratio of the polyoxyethylene unit in the total of the structural units of urethane resin A may be 1 mass% or higher and 50 mass% or lower. In this case, the film obtained from medical composition A can have excellent biocompatibility and strength. The content ratio of the polyoxyethylene unit in the total of the structural units of urethane resin A is preferably 2 mass% or higher, more preferably 3 mass% or higher, and still more preferably 5 mass% or higher. The content ratio of the polyoxyethylene unit in the total of the structural units of urethane resin A is also preferably 40 mass% or lower, more preferably 30 mass% or lower, and still more preferably 20 mass% or lower. A content ratio of the polyoxyethylene unit being 20 mass% or lower in the total of the structural units of urethane resin A is likely to lead to improved film strength and is likely to provide the film with the same moisture content as that of a urethane resin film not having a polyoxyethylene structure when the film is immersed in water.

The polyoxyethylene structure present in side chains of urethane resin A, even in a small amount, allows a film with excellent biocompatibility and excellent strength to form as compared with the polyoxyethylene structure present in the main chain, and is likely to limit an increase in the moisture content. Without wishing to be interpreted in a restrictive manner, this is considered likely due to the following: when urethane resin A comes into contact with an aqueous phase, the hydrophilic polyoxyethylene structures introduced into the hydrophobic polyurethane skeleton are rearranged so as to aggregate at the interface with the aqueous phase to the degree of freedom of the molecular chain, thereby allowing excellent biocompatibility to be brought about even in a small amount of polyoxyethylene structures introduced. In particular, given the ease of rearrangement of the polyoxyethylene structures occurring, whereas polyoxyethylene structures present in the main chain of the polymer have both ends of the polyoxyethylene fixed, polyoxyethylene structures present in side chains have only one end fixed, which allows for a greater degree of freedom of rearrangement, making it likely for biocompatibility to develop.

If polyoxyethylene structures are not present in the main chain of the polymer and present only in side chain portions, the film obtained from medical composition A tends to have a high degree of strength even when the film contains water.

The method for producing urethane resin A is not particularly limited. For example, a wide range of known methods for producing a urethane resin can be used. In an embodiment, the method for producing urethane resin A may include the step of subjecting the polyol and the isocyanate compound as described above to polymerization reaction to obtain urethane resin A. If the polyol and the polyisocyanate compound do not have a polyoxyethylene unit, polyethylene glycol (PEG) and/or branched polyethylene glycol (PEG) may be used as a starting material in the polymerization reaction. This yields urethane resin A with a polyoxyethylene structure.

The conditions of the polymerization reaction are not particularly limited, and may be, for example, the same conditions as those in the polymerization reaction of a known polyol and isocyanate compound. The temperature for the polymerization reaction may be, for example, about 30 to 130°C, and the reaction time may be about 30 minutes to 50 hours. In the polymerization reaction, a catalyst may be used, and examples of catalysts include an amine compound and a metal-based catalyst, such as tin octylate and bismuth octylate.

In the polymerization reaction, the proportion of the polyol and isocyanate compound for use is not particularly limited. For example, the molar ratio of isocyanate groups to hydroxyl groups may be 1 or greater, preferably 1.1 or greater, more preferably 1.2 or greater, and also preferably 3 or lower. This makes it likely for the emulsion of urethane resin A to be stable and to have a viscosity within an appropriate range.

The polymerization reaction may be performed in a solvent. The solvent for use may be selected from, for example, a wide range of organic solvents having no active hydrogen group that are commonly used in polyurethane synthesis, such as dioxane, methyl ethyl ketone, dimethylformamide, tetrahydrofuran, N-methyl-2-pyrrolidone, toluene, and propylene glycol monomethyl ether acetate.

For the polymerization reaction, polyethylene glycol (PEG) and/or branched polyethylene glycol (PEG) may be used. An example of polyethylene glycol for use is linear polyethylene glycol with a number average molecular weight of 100 to 10000, preferably 150 to 5000, and more preferably 200 to 3000. An example of branched polyethylene glycol is a polyol having a structure in which the hydrogen atom contained in an alkylene group having a hydroxyl group at each end is replaced with a group with a polyoxyethylene structure. Polyethylene glycol (PEG) and branched polyethylene glycol (PEG) for use may be obtained, for example, from commercial sources.

Using linear polyethylene glycol in the polymerization reaction yields urethane resin A having a polyoxyethylene structure in the main chain. Specifically, using linear polyethylene glycol yields urethane resin A having a polyoxyethylene structure in which each end of the linear polyethylene glycol is bound via a urethane bond. Using branched polyethylene glycol in the polymerization reaction may yield urethane resin A having a polyoxyethylene structure in side chains. In this case, the main chain of urethane resin A does not have a polyoxyethylene structure.

A urethane resin formed in the polymerization reaction of a polyol and an isocyanate compound has an isocyanate group in the polymer end, for example. Thus, the chain length can be further extended by using the terminal isocyanate group, as described later. A urethane resin having an isocyanate group at an end may be referred to as "urethane prepolymer." As described below, the urethane prepolymer can be dispersed in an aqueous phase to be emulsified.

The urethane prepolymer may have other structural units in addition to the structural unit derived from a polyol, the structural unit derived from an isocyanate compound, and the polyoxyethylene unit. The other structural units account for, for example, 10 mass% or lower, preferably 5 mass% or lower, and more preferably 1 mass% or lower of the urethane prepolymer.

Medical Composition B

Medical composition B contains a urethane resin. As described above, the urethane resin has a structural unit derived from a polycarbonate polyol and a structural unit derived from an aliphatic isocyanate, and also has at least one urea bond. The urethane resin is referred to below as "urethane resin B" contained in medical composition B.

Urethane resin B may have one, or two or more structural units derived from a polycarbonate polyol and one, or two or more structural units derived from an aliphatic isocyanate. The structural unit derived from a polycarbonate polyol can mean, for example, a group formed by removing the hydrogen atom from a hydroxyl group in a polycarbonate polyol. The structural unit derived from an aliphatic isocyanate can mean, for example, a group formed by adding a hydrogen atom to the nitrogen atom in an isocyanate group in an aliphatic isocyanate.

The polycarbonate polyol is not particularly limited, and can be selected from, for example, a wide variety of known polycarbonate polyols for forming urethane resins. Examples include polycarbonate polyols obtained by reacting a polyol compound with a carbonate compound. Examples of polyol compounds include aliphatic polyols and alicyclic polyols. Examples of carbonate compounds include carbonate derivatives, such as carbonate esters and phosgene.

The polycarbonate polyols are preferably those with a structure derived from an aliphatic polyol. Examples of aliphatic polyols include ethylene glycol, diethylene glycol, 1,4-butanediol, 1,3-butanediol, 2,3-butanediol, 1,3-propylene glycol, 1,2-propylene glycol, 1,6-hexanediol, 3-methyl-1,5-pentanediol, and neopentyl glycol.

The polycarbonate polyols may have one, or two or more structures derived from a polyol compound. The polycarbonate polyols may also have one, or two or more structures derived from a carbonate derivative.

The polycarbonate polyol preferably has two or more hydroxyl groups, and particularly preferably two hydroxyl groups.

The aliphatic isocyanates are not particularly limited and can be selected from, for example, a wide variety of known aliphatic isocyanates for forming urethane resins. Aliphatic isocyanates may be, for example, one, or two or more of the following: tetramethylene diisocyanate, dodecamethylene diisocyanate, hexamethylene diisocyanate (HMDI), 2,2,4-trimethylhexamethylene diisocyanate, 2,4,4-trimethylhexamethylene diisocyanate, lysine diisocyanate, 2-methylpentane-1,5-diisocyanate, and 3-methylpentane-1,5-diisocyanate.

The aliphatic isocyanate preferably has two or more isocyanate groups, and particularly preferably two isocyanate groups.

Due to urethane resin B having both a structural unit derived from a polycarbonate polyol and a structural unit derived from an aliphatic isocyanate, the film obtained from medical composition B particularly has excellent strength in addition to being able to easily inhibit non-specific adsorption of proteins and adsorption of platelets.

Urethane resin B has a urea bond. The urea bond can be formed by extending the chain length with a chain extender from the isocyanate group at an end of the urethane prepolymer described later as the base point.

Urethane resin B may have a polyoxyethylene structure in the main chain and/or side chains. Specifically, urethane resin B may have a polyoxyethylene unit at least at either the main chain or side chains, or at both the main chain and side chains. The polyoxyethylene structure is present by being covalently bound to at least at either the main chain or side chains, or both, of urethane resin B.

For urethane resin B, the number average molecular weight of the polyoxyethylene unit is not particularly limited and may be, for example, 100 to 10000, preferably 150 to 5000, and more preferably 200 to 3000. The number average molecular weight of the polyoxyethylene unit referred to in the present specification means a value as measured by GPC.

As described later, urethane resin B with a polyoxyethylene structure can be obtained, for example, by using polyethylene glycol (PEG) or branched polyethylene glycol (PEG) as a starting material during polymerization reaction of urethane resin. Alternatively, urethane resin B with a polyoxyethylene structure can be obtained by reacting urethane resin with polyethylene glycol (PEG) or branched polyethylene glycol (PEG).

If urethane resin B has a polyoxyethylene structure, the content ratio of the polyoxyethylene unit is not particularly limited. The content ratio of the polyoxyethylene unit in the total of the structural units of urethane resin B may be 50 mass% or lower. In this case, the film obtained from medical composition B can have excellent biocompatibility and strength. The content ratio of the polyoxyethylene unit in the total of the structural units of urethane resin B is preferably 0.1 mass% or higher, more preferably 1 mass% or higher, and still more preferably 3 mass% or higher. The content ratio of the polyoxyethylene unit in the total of the structural units of urethane resin B is also preferably 40 mass% or lower, more preferably 30 mass% or lower, and still more preferably 20 mass% or lower.

The polyoxyethylene structure present in side chains of urethane resin B, even in a small amount, allows a film with excellent biocompatibility and excellent strength to form as compared with the polyoxyethylene structure present in the main chain, and is likely to limit an increase in the moisture content. This is considered likely due to the same reason inferred in the case of urethane resin A, discussed above.

The method for producing urethane resin B is not particularly limited. For example, a wide range of known methods for producing a urethane resin can be used. In an embodiment, the method for producing urethane resin B may include the step of subjecting a polycarbonate polyol and an aliphatic isocyanate to polymerization reaction to obtain urethane resin B.

The conditions of the polymerization reaction are not particularly limited, and may be, for example, the same conditions as those in the polymerization reaction of a known polycarbonate polyol and aliphatic isocyanate. The temperature for the polymerization reaction may be, for example, about 30 to 130°C, and the reaction time may be about 30 minutes to 50 hours. In the polymerization reaction, a catalyst may be used, and examples of catalysts include an amine compound and a metal-based catalyst, such as tin octylate and bismuth octylate.

In the polymerization reaction, the proportion of the polycarbonate polyol and aliphatic isocyanate for use is not particularly limited. For example, the molar ratio of isocyanate groups to hydroxyl groups may be 1 or greater, preferably 1.1 or greater, more preferably 1.2 or greater, and also preferably 3 or lower. This makes it likely for the emulsion of urethane resin B to be stable and to have a viscosity within an appropriate range.

The polymerization reaction may be performed in a solvent. The solvent for use may be selected from, for example, a wide range of organic solvents having no active hydrogen group that are commonly used in polyurethane synthesis, such as dioxane, methyl ethyl ketone, dimethylformamide, tetrahydrofuran, N-methyl-2-pyrrolidone, toluene, and propylene glycol monomethyl ether acetate.

For the polymerization reaction, polyethylene glycol (PEG) and/or branched polyethylene glycol (PEG) may be used. An example of polyethylene glycol for use is linear polyethylene glycol with a number average molecular weight of 100 to 10000, preferably 150 to 5000, and more preferably 200 to 3000. An example of branched polyethylene glycol is a polyol having a structure in which the hydrogen atom contained in an alkylene group having a hydroxyl group at each end is replaced with a group with a polyoxyethylene structure. Polyethylene glycol (PEG) and branched polyethylene glycol (PEG) for use may be obtained, for example, from commercial sources.

Using linear polyethylene glycol in the polymerization reaction yields urethane resin B having a polyoxyethylene structure in the main chain. Specifically, using linear polyethylene glycol yields urethane resin B having a polyoxyethylene structure in which each end of the linear polyethylene glycol is bound via a urethane bond. Using branched polyethylene glycol in the polymerization reaction may yield urethane resin B having a polyoxyethylene structure in side chains. In this case, the main chain of urethane resin B does not have a polyoxyethylene structure.

A urethane resin formed in the polymerization reaction of a polycarbonate polyol and an aliphatic isocyanate has an isocyanate group in the polymer end, for example. Urethane resin B is formed by further extending the chain length from the terminal isocyanate group. This elongation of the chain length forms urea bonds in urethane resin B. A urethane resin having an isocyanate group at an end (i.e., urethane prepolymer) can be dispersed in an aqueous phase to be emulsified, as described later. After this emulsification, the urethane prepolymer can be reacted with a chain extender to obtain urethane resin B having urea bonds formed.

The urethane prepolymer may have other structural units in addition to the structural unit derived from a polycarbonate polyol, the structural unit derived from an aliphatic isocyanate, and the optional polyoxyethylene unit. The other structural units account for, for example, 10 mass% or lower, preferably 5 mass% or lower, and more preferably 1 mass% or lower of the urethane prepolymer.

Aqueous Dispersion

As described above, the medical composition according to the present invention includes medical composition A and medical composition B. The form of the medical composition according to the present invention is not particularly limited, but is preferably an aqueous dispersion.

The method for producing the aqueous dispersion is not particularly limited. For example, a wide range of known methods for producing a urethane resin dispersion can be used. For example, an aqueous dispersion can be obtained by emulsifying the urethane prepolymer described above in an aqueous phase (i.e., water). In this emulsification, the chain extender allowed to be present together elongates the chain length of the urethane prepolymer, thereby increasing the molecular weight of the urethane resin. The urethane resin obtained by increasing the chain length of the urethane prepolymer with a chain extender has urea bonds formed.

The chain extender is not particularly limited; specifically, a known compound for use in extending the chain length of a urethane prepolymer can be used. Examples of chain extenders include polyamines, such as ethylene diamine, trimethylene diamine, piperazine, isophorone diamine, diethylenetriamine, dipropylene triamine, and triethylenetetramine; and polyols, such as ethylene glycol, diethylene glycol, 1,4-butanediol, 1,3-butanediol, 2,3-butanediol, 1,3-propylene glycol, 1,2-propylene glycol, 1,6-hexanediol, 3-methyl-1,5-pentanediol, and neopentyl glycol.

The chain extender may also be a water molecule. Thus, the molecular weight of the urethane prepolymer can be increased by water molecules present in the aqueous phase, which is a dispersion medium used in the emulsification described above. A chain extender with multi-functionality, such as diethylenetriamine, can form a urethane resin that is flexible yet tough because such a chain extender can provide the urethane resin with a three-dimensional network structure, which gives flexibility.

The emulsification of the urethane prepolymer described above may also be performed by using a surfactant. The surfactant is not particularly limited, and examples include nonionic surfactants, anionic surfactants, and cationic surfactants. These surfactants may be used singly, or in a combination of two or more.

Examples of nonionic surfactants include alcohols having 8 to 24 carbon atoms, alkenols having 8 to 24 carbon atoms, polycyclic phenols, amines having 8 to 44 carbon atoms, amides having 8 to 44 carbon atoms, fatty acids having 8 to 24 carbon atoms, polyhydric alcohol esters of fatty acids, fats and oils, alkylene oxide adducts of polypropylene glycol, alkylene oxide adducts of polycyclic phenols, and Pluronic (registered trademark) nonionic surfactants. Examples of alkylene oxide adducts of polycyclic phenols include polyoxyethylene distyrylphenyl ether-form nonionic surfactants, polyoxyethylene polyoxypropylene distyrylphenyl ether-form nonionic surfactants, polyoxyethylene tristyrylphenyl ether-form nonionic surfactants, and polyoxyethylene polyoxypropylene tristyrylphenyl ether-form nonionic surfactants. If two or more alkylene oxides are added in a nonionic surfactant, the addition may be block addition or random addition.

Examples of anionic surfactants include anionized products of alcohols, alkenols, or alkylene oxide adducts of the nonionic surfactants as described above. Examples of cationic surfactants include monoalkyl trimethyl ammonium salts having 8 to 24 carbon atoms, dialkyl dimethyl ammonium salts having 8 to 24 carbon atoms, monoalkyl amine acetate having 8 to 24 carbon atoms, dialkyl amine acetate having 8 to 24 carbon atoms, and quaternary salts of alkyl imidazoline having 8 to 24 carbon atoms.

From the viewpoint of excellent miscibility with other components, the surfactant is preferably a nonionic surfactant, and more preferably an alkylene oxide adduct of a polycyclic phenol or a Pluronic (registered trademark)-form nonionic surfactant.

The amount of the surfactant for use is preferably 0.5 parts by mass or more, more preferably 2 parts by mass or more, and preferably 10 parts by mass or less, more preferably 8 parts by mass or less, per 100 parts by mass of the urethane prepolymer for use.

As described above, emulsification of the urethane prepolymer, and optional chain length elongation (i.e., an increase in the molecular weight), yield a urethane resin dispersed in water (an aqueous dispersion of polyurethane). The average particle size of the urethane resin in an aqueous dispersion is not particularly limited and can be the same as that of known aqueous dispersions of polyurethane. For example, the average particle size of the urethane resin is 0.01 to 50 pm, preferably 0.015 to 10 pm, and more preferably 0.02 to 5 um. The average particle size of the urethane resin is a 50% cumulative value as determined by dynamic light scattering, which can be measured by using, for example, a Microtrac UPA particle size analyzer (Model No. 9340, manufactured by Nikkiso Co., Ltd).

The urethane resin (urethane resin A and urethane resin B) in the form of fine particles can be stably dispersed in water, for example, due to a hydrophilic component present in the polymer chain, and can form an aqueous dispersion as an "aqueous polyurethane resin." If the urethane resin has a polyoxyethylene moiety, as in urethane resin A, the moiety can function as a hydrophilic segment in an aqueous phase.

The medical composition in the form of an aqueous dispersion can be used to form a urethane resin-containing film on various substrates. Thus, if the medical composition is in the form of an aqueous dispersion, the aqueous dispersion may be used as a coating agent. An aqueous dispersion can be applied to the surface of a substrate without containing an organic solvent, and can thus be used for substrates that are less resistant to organic solvents.

A film formed from the medical composition (e.g., a film formed from the aqueous dispersion descried above) contains the urethane resin described above (specifically, urethane resin A or urethane resin B described above) and exhibits high biocompatibility.

In the present specification, the term "biocompatibility" refers to properties of being less likely to be recognized as a foreign substance in contact with a biological substance or a substance of biological origin, such as inhibiting the non-specific adsorption of proteins or the adhesion frequency of platelets. Specifically, the term means, for example, not causing complement activation or platelet activation and being minimally invasive or noninvasive to tissue. The term "biocompatibility" also includes the embodiment of "hemocompatibility"; the term "hemocompatibility" mainly means not causing blood coagulation due to adhesion or activation of platelets.

It is presumed that when the urethane resin is urethane resin A or urethane resin B with polyoxyethylene structures, due to the highly hydrophilic polyoxyethylene structures introduced into the hydrophobic polyurethane skeleton, the polymer that has come into contact with an aqueous phase undergoes rearrangement such that the polyoxyethylene structures aggregate on the polymer surface in order to relax interfacial energy inside the polymer. Thus, it is presumed that medical composition A, containing urethane resin A, is more likely to exhibit properties such as biocompatibility due to the polyoxyethylene structures, and that the properties are more pronounced in the form of an aqueous dispersion. The same is true for medical composition B containing urethane resin B that has polyoxyethylene structures.

It is now clear that the development of biocompatibility is due to molecular mobility of polyoxyethylene structures and that the molecular mobility is affected by, for example, the surrounding polar functional groups. Given these facts, it can be inferred that the reason why urethane resin exhibits biocompatibility when containing polyoxyethylene structures (PEG) is at least because the molecular mobility of polyoxyethylene structures is interfered with by urethane bonds to a less extent. Thus, the degree of biocompatibility can be adjusted by adjusting the content ratio of polyoxyethylene structures in urethane resin or by designing the position for introducing polyoxyethylene structures.

When the urethane resin is urethane resin A or urethane resin B that has polyoxyethylene structures, development of particularly remarkable biocompatibility is observed, such as inhibition of non-specific adsorption of proteins and inhibition of adhesion frequency of platelets at a level equal to or higher than that of PMEA (poly-2-methoxyethylacrylate), which is a widely used biocompatible polymer, and polymers containing MPC (2-methacryloyloxyethylphosphorylcholine) units (e.g., an MPC polymer, which is a copolymer of MPC and BMA, described later).

Because PEG is not necessarily highly chemically stable, there have been few options for immobilizing PEG and making PEG water-insoluble; polymer compositions that can be widely used in various substrates as a coating agent that exhibits biocompatibility as well as water solubility resistance have not necessarily been obtained.

In particular, a water-insoluble polymer formed by incorporating PEG in some form into part of the structure of another polymer for use as a coating agent generally must be dissolved in some sort of organic solvent to be applied to a substrate. Thus, in the context of the material of the substrate to be coated, an organic solvent that can dissolve the polymer while not damaging the substrate should be available. In this respect, there has been difficulty in obtaining polymer compositions that are usable for a wide range of substrates while having desired properties.

In response, the inventors found that the introduction of polyoxyethylene structures into urethane resin, as in medical composition A, produces a phenomenon that supports the presence of biocompatibility, such as exhibiting properties such as water solubility resistance while maintaining the biocompatibility exhibited by PEG and inhibiting the nonspecific adsorption of proteins and the adhesion frequency of platelets. The inventors also found that the same phenomenon occurs in medical composition B when urethane resin B has polyoxyethylene structures.

Due to highly hydrophilic polyoxyethylene structures introduced into a polyurethane skeleton exhibiting hydrophobicity, properties such as biocompatibility originating from the polyoxyethylene structures can appear. If a hydrophobic structure and a hydrophilic structure are adjacent to each other, a high level of interfacial energy generally forms between them. Thus, there is presumably a tendency to relax the interfacial energy inside the polymer by rearrangement of the structures occurring within the degree of freedom possessed by the molecular chain.

It is presumed that when the urethane resin has polyoxyethylene structures, contact of a polyurethane film with an aqueous phase causes rearrangement such that the polyoxyethylene structures introduced into the polyurethane skeleton aggregate on the surface of the polyurethane film by using the energy at the interface between the film and the aqueous phase as a driving force. This appears to indicate that a polyurethane skeleton is preferred as a skeleton for use in making polyoxyethylene structures non-water-soluble. It is speculated that the polyoxyethylene structure moieties present on the surface of a polyurethane film that comes in contact with an aqueous phase come into the state referred to as, for example, "polymer brushes," which cover the polyurethane skeleton, thereby preventing the non-specific adsorption of proteins.

In the present invention, the urethane resin does not necessarily have to have a polyoxyethylene structure, as in urethane resin B contained in medical composition B. Medical composition B, containing urethane resin B, can form a film that exhibits biocompatibility without having polyoxyethylene structures, and the film can inhibit non-specific adsorption of proteins or reduce the adhesion frequency of platelets (i.e., exhibiting antithrombogenicity).

Urethane resin B that does not have polyoxyethylene structures allows the film to have a significantly higher degree of strength and also to reduce the adhesion frequency of platelets as compared with urethane resin B that has polyoxyethylene structures. Additionally, urethane resin B that does not have polyoxyethylene structures is highly water resistant because the moisture content of the film can be low. Thus, urethane resin B that does not have polyoxyethylene structures exhibits high dimensional stability even when used in a wet environment and is advantageous in practical use. Urethane resin B that has polyethylene structures exhibits further improved biocompatibility and is likely to exhibit improved dispersibility in water.

As described above, the medical composition according to the present invention (specifically medical composition A and medical composition B) is suitable for medical applications described below due to its ability to form a film excellent in biocompatibility. Because the film formed from the medical composition according to the present invention contains a specific urethane resin (urethane resin A, urethane resin B), the intermediate water mentioned above is presumably formed in the film. Without wishing to be interpreted in a restrictive manner, the excellent biocompatibility described above can appear due to the intermediate water present in the film.

## 2. Method of Use and Application of Medical Composition

The medical composition of the present invention, which contains a urethane resin having high biocompatibility (specifically, urethane resin A or urethane resin B described above), is suitable for use in various medical applications.

The medical composition of the present invention can be used to form a film. Such a film can be formed, for example, on various substrates, such as various medical instruments and artificial organs, or can be formed, for example, on various medical fibers. When the medical composition of the present invention is used to form a film, the medical composition is an aqueous dispersion as described above.

Since a medical instrument comprising a film formed of the medical composition of the present invention contains a urethane resin, biocompatibility can be imparted to the medical instrument or artificial organ. That is, a medical instrument or an artificial organ comprising the film formed of the medical composition of the present invention is inhibited from non-specific adsorption of proteins or adhesion frequency of platelets.

Examples of the "artificial organ" and the "medical equipment" include, but are not limited to, artificial organs and medical equipment having a portion in contact with a biological material, such as blood. Specific examples include, but are not limited to, blood filters, artificial lung devices, dialysis devices, blood storage bags, platelet storage bags, blood circuits, artificial hearts, indwelling needles, catheters, guide wires, stents, artificial blood vessels, and endoscopes.

The material and shape of the substrate of the "artificial organ" and "medical equipment" are not particularly limited. Examples of the material include natural polymers, such as cotton and hemp; and synthetic polymers, such as nylon, polyester, polyacrylonitrile, polyolefin, halogenated polyolefin, polyurethane, polyamide, polycarbonate, polysulfone, polyethersulfone, poly(meth)acrylate, ethylene-vinyl alcohol copolymers, and butadiene-acrylonitrile copolymers; and mixtures thereof. Examples also include metals, ceramics, and composite materials thereof. The artificial organ and medical equipment may be composed of multiple kinds of substrates. Examples of the shape of the substrate include porous materials, fibers, nonwoven fabrics, particles, films, sheets, tubes, hollow fibers, powders, and the like.

When anti-bacterial adhesion properties and/or antiinflammatory properties are imparted to, for example, an artificial organ or a medical equipment, the film is preferably formed on at least a part of the surface in contact with in vivo tissue or blood, and more preferably almost the entire surface in contact with in vivo tissue or blood.

The medical composition of the present invention can be used as a material that constitutes the entire artificial organ or medical equipment used in contact with in vivo tissue or blood, or as a material that constitutes the surface portion thereof. Preferably, at least a part of the surface in contact with blood, and more preferably almost the entire surface in contact with blood, of a medical equipment is composed of a film formed of the medical composition of the present invention. Examples of such medical equipment include implantable artificial organs and therapeutic devices, extracorporeal circulatory artificial organs, surgical sutures, catheters (angiographic catheters, guidewires, circulatory catheters, such as PTCA catheters, digestive catheters, such as gastric catheters, gastrointestinal catheters, and esophageal tubes, tubes, and urological catheters, such as urinary catheters and urethral catheters). Further, the film can also be used as a hemostatic agent, an adhesive material for biological tissue, a repair material for tissue regeneration, a carrier for a drug sustained-release system, a hybrid artificial organ, such as an artificial pancreas and an artificial liver, an artificial blood vessel, an embolic material, a matrix material for a scaffold for cellular engineering, or the like. Further, surface lubricity may be imparted to these artificial organs and medical equipment in order to facilitate their insertion into the blood or tissue without damaging the tissue.

At least a part of the surfaces of the substrate that constitutes a blood filter may be coated with the medical composition of the present invention. At least a part of the blood-contacting surfaces of a blood bag and a tube communicating with the blood bag may be coated with the polymer composition of the present invention. At least part of the blood-contacting surfaces of the extracorporeal circulation blood circuit composed of an equipment-side blood circuit section composed of a tube, an arterial filter, a centrifugal pump, a hemoconcentrator, a cardioplegia, and the like, and an operation-field-side blood circuit section composed of a tube, a catheter, a sucker, and the like may be coated with the medical composition of the present invention.

When the medical composition of the present invention is used for an indwelling needle assembly, at least a part of the blood-contacting surfaces of the indwelling needle assembly may be coated with the medical composition of the present invention, the indwelling needle assembly comprising an inner needle having a sharp needle tip at the tip, an inner needle hub installed on the proximal side of the inner needle, a hollow outer needle into which the inner needle can be inserted, an outer needle hub installed on the proximal side of the outer needle, a protector mounted on the inner needle and movable in the axial direction of the inner needle, and a connecting means for connecting the outer needle hub and the protector. Further, at least a part of the blood-contacting surfaces of a catheter composed of a long tube and an adapter connected to the proximal end (proximal side) of the long tube may be coated with the medical composition of the present invention.

At least a part of the blood-contacting surfaces of the guide wire may be coated with the medical composition of the present invention. Further, at least a part of the blood-contacting surface of stents of various shapes, such as hollow tubular bodies made of a metal material or a polymer material with pores formed on a lateral side thereof, or those formed by knitting metal wires or polymer fibers into cylindrical shapes, may be coated with the medical composition of the present invention.

When the medical composition of the present invention is used for an artificial heart-lung machine, the polymer composition of the present invention may be applied to the outer surface or the outer surface layer of the hollow fiber membrane of a hollow fiber membrane external blood perfusion-type artificial lung machine in which a large number of porous hollow fiber membranes for gas exchange are housed in a housing, blood flows to the outer surface side of the hollow fiber membrane, and an oxygen-containing gas flows inside the hollow fiber membrane.

The medical equipment may be a dialysis device comprising a dialysate circuit comprising at least one dialysate container filled with a dialysate and at least one drainage container for collecting the dialysate, and a liquid delivery means for delivering the dialysis fluid from the dialysis fluid container as the starting point to the drainage container as the ending point, wherein at least a part of the surface in contact with blood may be coated with the medical composition of the present invention.

Examples of the method for maintaining the film of the medical composition of the present invention on the surface of the artificial organ or medical equipment include coating by a usual application method and other known methods, such as graft polymerization by irradiation, electron beams, or ultraviolet rays, and introduction using a chemical reaction with a functional group of a substrate. Among these, the coating method is preferred for practical use because of its ease of production operation. Further, examples of the coating method include, but are not limited to, application, spraying, dipping, and like methods, which can be suitably selected and used according to the purpose. The thickness of the film formed of the polymer composition of the present invention is not particularly limited. For example, the polymer composition can be formed into a film with a thickness of about 0.1 $\mu$m to about 1 mm and used.

The coating treatment by the method of applying the medical composition of the present invention can be carried out by using the medical composition of the present invention or a simple operation, such as immersing a member to be coated in a solution formed by dissolving a composition containing the biocompatible polymer composition of the present invention in a suitable solvent, then removing excess solution, and subsequently air-drying the member. Further, in order to more firmly immobilize the medical composition of the present invention to the member to be coated, heat may be applied after coating to further enhance the adhesion of the member to the medical composition of the present invention. Alternatively, the surface may be cross-linked for immobilization. The crosslinking method may be, for example, introduction of a crosslinkable monomer as a comonomer component. Electron beams, $\gamma$-rays, or light irradiation may be used for crosslinking.

Taking advantage of the fact that non-specific adsorption of proteins to the polyurethane surface formed of the medical composition of the invention and subsequent denaturation and multilayer adsorption can be inhibited, the medical composition of the present invention can be used to form an antifouling surface to prevent various types of biological contamination by reducing bacterial adhesion frequency. That is, it is known that when *E. coli* or the like adheres to a material surface, a protein or the like adsorbed to the material surface is used as a scaffold. On a surface where protein adsorption frequency is low, such as the surface of the medical composition of the present invention, the frequency of *E. coli* adhesion is expected to be reduced.

As applications of forming an antifouling surface, adsorption of various proteins and bacteria contained in water can be expected to be inhibited by applying the medical composition of the present invention to the surface that comes into contact with water containing the various proteins and bacteria, such as general sinks, baths, and toilets, as well as sinks for washing various instruments and dirty materials using water at medical sites. The antifouling material can be used not only as a material for preventing infectious diseases or a material for preventing bacterial adhesion, but also as a biofouling prevention material for preventing the adhesion of organisms by being used in portions that come into contact with water in which organisms are alive, such as ship bottoms and seawalls, and where the adhesion of various organisms becomes a problem.

As described above, excellent biocompatibility can be imparted to a medical instrument or the like by forming a film of the medical composition of the present invention (in particular, the aqueous dispersion) on the medical instrument or the like. Accordingly, the step of forming a film of the medical composition of the present invention on a medical instrument is suitable for a protein adsorption inhibition method or a platelet adsorption inhibition method. Further, the step of treating medical fibers with the medical composition of the present invention is suitable as a method for treating medical fibers.

The medical composition of the present invention is suitable for use in various applications, such as a protein adsorption inhibitor, a platelet adsorption inhibitor, and a medical fiber treatment agent.

The coating agent, protein adsorption inhibitor, platelet adsorption inhibitor, and medical fiber treatment agent may contain other components as long as they contain the medical composition of the present invention. Alternatively, the coating agent, protein adsorption inhibitor, platelet adsorption inhibitor, and medical fiber treatment agent may consist only of the medical composition of the present invention. The method for preparing the coating agent, the protein adsorption inhibitor, the platelet adsorption inhibitor, and the medical fiber treatment agent containing the medical composition of the present invention is not particularly limited, and the coating agent, the protein adsorption inhibitor, the platelet adsorption inhibitor, and the medical fiber treatment agent containing the medical composition of the present invention can be prepared by an appropriate method using the medical composition of the present invention.

Examples

The following describes the present invention in more detail with reference to Examples. However, the present invention is not limited to the embodiments of these Examples.

Preparation and Evaluation of Medical Composition A

Synthesis of Aqueous Polyurethane Dispersion

Example 1

An aqueous polyurethane dispersion was prepared according to the kind and proportion (parts by mass) of each starting material component shown in Table 1. Using a four-necked flask equipped with a stirrer, a reflux condenser, a thermometer, and a nitrogen-feeding tube, 49.9 parts by mass of polycarbonate diol of 1,6-hexanediol (PCD (1,6-HD), produced by Ube Corporation, product name: ETERNACOLL UH-100, number average molecular weight: about 1000, average hydroxyl value: 110 mg KOH/g) as a polyol component (component A), 33.3 parts by mass of polyethylene glycol (product name: PEG-1000, produced by DKS Co. Ltd., product name: PEG-1000, number average molecular weight: about 1000, average hydroxyl value: 110 mg KOH/g)), 16.8 parts by mass of hexamethylene diisocyanate (HMDI) (Duranate 50MS, produced by Asahi Kasei Chemicals Corporation) as a polyisocyanate component (component B), and 0.0035 parts by mass of an organotin compound (bis(neodecanoyloxy)dioctylstannane, produced by Songwon Industrial Co., Ltd.) as a reaction catalyst were added to 90 parts by mass of methyl ethyl ketone as a reaction medium. The resulting mixture was allowed to react at 75°C for 4 hours to produce a urethane prepolymer, and a solution of the urethane prepolymer in methyl ethyl ketone was obtained.

The solution was cooled to 45°C. While 1408 parts by mass of water was gradually added to a mixture of a surfactant in an amount equivalent to 6.0 parts by mass (product name: Noigen EA-157, produced by DKS Co. Ltd.) and a silicone antifoaming agent in an amount equivalent to 0.05 parts by mass, a urethane prepolymer was emulsified and dispersed in a liquid phase using a homogenizer to obtain an aqueous dispersion. Then, after a chain extension reaction with water was carried out for 1 hour, the reaction mixture was maintained in an environment of 50°C under reduced pressure and methyl ethyl ketone as a reaction medium was removed by evaporation to obtain an aqueous polyurethane dispersion containing about 7.3% of a non-volatile component.

Examples 2 to 10

As shown in Table 1, Aqueous polyurethane dispersions were prepared in the same manner as in Example 1 except that the proportions of PCD (1,6-HD) and HMDI, and the proportion, the average molecular weight, the structure, etc. of PEG to be used were varied.

Note that PEG 600 (produced by DKS Co. Ltd., product name: PEG 600S, number average molecular weight: about 600, average hydroxyl value: 187 mg KOH/g), and PEG 2000 (produced by DKS Co. Ltd., product name: PEG 2000, number average molecular weight: about 2000, average hydroxyl value: 56 mg KOH/g) in Table 1 are polyethylene glycols. Further, the branched PEG is a diol (Ymer N120, produced by Perstorp Polyol Inc.) having a structure in which hydrogen atoms of an alkylene chain having hydroxyl groups at both ends are replaced with, for example, a group containing a polyoxyethylene structure, and has a number average molecular weight of about 1020 and an average hydroxyl value of 110 mg KOH/g.

Table 1

| Starting materials | | | Examples | | | | | | | | | |
|---|---|---|---|---|---|---|---|---|---|---|---|---|
| | | | 1 | 2 | 3 | 4 | 5 | 6 | 7 | 8 | 9 | 10 |
| Component A | PCD (1,6-HD) | Mw = 1000 | 49.9 | 34.5 | 57.6 | 37.5 | 49.6 | 76.5 | 80.2 | 71.6 | 76.6 | 80.3 |
| Component B | HMDI | Mw = 168.19 | 16.8 | 16.3 | 19.4 | 12.6 | 16.7 | 16.8 | 16.8 | 16.8 | 16.8 | 16.8 |
| PEG | PEG-1000 | Mw = 1000 | 33.3 | 49.2 | | | | | | 11.6 | 6.7 | 2.9 |
| | PEG-600 | Mw = 600 | | | 23.0 | | | | | | | |
| | PEG-2000 | Mw = 2000 | | | | 49.9 | | | | | | |
| | Branched PEG | Mw = 1020 | | | | | 33.7 | 6.8 | 3.0 | | | |
| Reaction catalyst | | | 0.0035 | 0.003 | 0.0015 | 0.003 | 0.003 | 0.002 | 0.002 | 0.002 | 0.002 | 0.002 |
| Surfactant | | | 6 | 6 | 10 | 6 | 0 | 10 | 10 | 11 | 11 | 10 |
| Defoaming agent | | | 0.05 | 0.1 | 0.01 | 0.1 | 0.01 | 0.01 | 0.01 | 0.01 | 0.01 | 0.01 |
| Water | | | 1408 | 1408 | 1408 | 1408 | 400 | 400 | 400 | 629 | 629 | 629 |

Examples 11 to 17

Aqueous polyurethane dispersions were prepared in the same manner as in Example 1 except that the kinds and proportions of the polyol component (component A) and the polyisocyanate component (component B) and the proportion, the average molecular weight, the structure, etc., of PEG to be used were varied in accordance with the kind and proportion (parts by mass) of each starting material component shown in Table 2.

Note that in Table 2, the polyester polyol (MPD/AA; 3-methyl-1,5-pentanediol/adipic acid) in Table 2 is a polyester polyol having a number average molecular weight of about 1000 (Kurapole P-1010, produced by Kuraray Co., Ltd.), and the polytetramethylene glycol (PTMG) is a polytetramethylene glycol (PTMG) having a number average molecular weight of about 1000 (PloyTHF 1000S: produced by BASF Japan Ltd.).

Table 2

| Starting materials | | | Examples | | | | | | |
|---|---|---|---|---|---|---|---|---|---|
| | | | 11 | 12 | 13 | 14 | 15 | 16 | 17 |
| Com ponent A | PCD (1,6-HD) | Mw = 1000 | 64.8 | 45.4 | 55.8 | | | 47.4 | 49.6 |
| | MPD/AA | Mw = 1000 | | | | 49.9 | | | |
| | PTMG | Mw = 1000 | | | | | 49.9 | | |
| | TMP | Mw = 134.17 | 1.8 | | | | | | |
| | MPD | Mw = 118.17 | | | 4.8 | | | | |
| Com ponent B | HMDI | Mw = 168.19 | 19.8 | | 22.1 | 16.8 | 16.8 | | |
| | H12MDI | Mw = 262.35 | | 23.8 | | | | | |
| | IPDI | Mw = 222.29 | | | | | | 21.1 | |
| | TDI | Mw = 174.16 | | | | | | | 17.3 |
| PEG | PEG-1000 | Mw = 1000 | | | | 33.3 | 33.3 | 31.6 | 33.1 |
| | Branched PEG | Mw = 1020 | 13.5 | 30.9 | 17.3 | | | | |
| Reaction catalyst | | | 0.005 | 0.05 | 0.002 | 0.002 | 0.006 | 0.022 | 0.022 |
| Surfactant | | | 10 | 0 | 10 | 6 | 6 | 6 | 6 |
| Defoaming agent | | | 0.01 | 0.01 | 0.01 | 0.1 | 0.05 | 0.01 | 0.05 |
| Water | | | 400 | 400 | 400 | 300 | 300 | 300 | 300 |
| MPD/AA: polyester polyol (3-methyl-1,5-pentanediol/adipic acid)<br>PTMG: polytetramethylene glycol<br>TMP: trimethylolpropane<br>MPD: 3-methyl-1,5-pentanediol<br>H12MDI: dicyclohexylmethane diisocyanate<br>HMDI: hexamethylene diisocyanate<br>IPDI: isopholone diisocyanate<br>TDI: tolylene diisocyanate | | | | | | | | | |

Tables 3 and 4 show the results of measuring the free isocyanate group content relative to the non-volatile matter in the urethane prepolymer solutions of methyl ethyl ketone synthesized in Examples 1 to 17 in accordance with JISK 7301. Further, Tables 3 and 4 also show the results of measuring the weight of the non-volatile matter in each aqueous polyurethane dispersion in accordance with JIS K 6828-1: 2003 (weight of the non-volatile matter of the aqueous polyurethane dispersion).

Table 3

|  | 1 | 2 | 3 | 4 | 5 | 6 | 7 | 8 | 9 | 10 |
|---|---|---|---|---|---|---|---|---|---|---|
| Number of free NCO groups (%) | 1.40 | 1.10 | 1.61 | 1.05 | 1.39 | 1.40 | 1.39 | 1.40 | 1.40 | 1.40 |
| Non-volatile matter (%) | 7.3 | 9.6 | 15.4 | 12.7 | 21.7 | 29.5 | 24.1 | 18.1 | 16.8 | 23.3 |

Table 4

|  | Examples | | | | | | |
|---|---|---|---|---|---|---|---|
|  | 11 | 12 | 13 | 14 | 15 | 16 | 17 |
| Number of free NCO groups (%) | 1.67 | 1.27 | 1.54 | 1.40 | 1.40 | 1.33 | 1.39 |
| Non-volatile matter (%) | 22.2 | 22.8 | 23.5 | - | - | - | - |

Evaluation of Protein Adsorption

The polymer compositions obtained in Examples 5 and 13 were evaluated for their protein (fibrinogen) adsorption by the following method.

In order to form a polyurethane film of each of the above polymer compositions on a 96-well plate made of polypropylene by using the polymer composition, an aqueous dispersion of each polymer composition was freeze-dried to isolate each polymer, and then dissolved in dichloromethane ($CH_2Cl_2$) to produce a 0.2 (wt/v%) solution. The obtained solution was added dropwise into a 96-well plate and dried to form each polyurethane film on the bottom of the 96-well plate, and was used as a sample. For comparison, PP (polypropylene), which does not show biocompatibility, PMEA, which is known to show high biocompatibility, and a polymer produced by copolymerizing MPC (2-methacryloyloxyethyl phosphorylcholine) and BMA (butyl methacrylate) and making the copolymer insoluble in water (this polymer is indicated as "MPC polymer" in the Examples) were each formed into a film in the same manner, and the resulting products were used as samples. The MPC polymer had a molar ratio of MPC:BMA of 3:7.

An aqueous solution adjusted so that the amount of fibrinogen per unit area was 156 ($\mu$g/cm$^2$) was supplied to each of the above samples. In this state, each sample was incubated at 37°C for 1 hour and then the aqueous solution was removed and the wells were washed with PBS (-). In order to collect the fibrinogen adsorbed on each well in the aqueous phase, 30 $\mu$L of 0.5% SDS + 1N NaOH aqueous solution was added and the resulting mixture was incubated for 2 hours. The protein was quantified by using a Micro BCA Protein Assay Kit (produced by Thermo Fisher Scientific Inc.).

Table 5 shows the measurement results. It was observed that while 3.8 $\mu$g/cm$^2$ of fibrinogen was adsorbed on the surface of PP, which does not show biocompatibility, 0.8 $\mu$g/cm$^2$ and 1.7 $\mu$g/cm$^2$ of fibrinogen were adsorbed on the surfaces of PMEA and MPC polymers, respectively, which are known to show biocompatibility.

On the other hand, the polyurethane films of the present invention obtained in Examples 5 ad 13 were observed to adsorb fibrinogen in an amount of 1.7 $\mu$g/cm$^2$ and 0.8 $\mu$g/cm$^2$, respectively. The results thus showed that protein adsorption to the surface of the polyurethane films of Examples 5 and 13 was equivalent to that of PMEA and MPC polymers.

The above results show that adsorption of proteins can be inhibited by using a urethane resin having a urethane bond in the main chain and having a polyoxyethylene structure in the main chain and/or a side chain thereof.

Table 5

|  | Example 5 | Example 13 | PP | PMEA | MPC polymer |
|---|---|---|---|---|---|
| Fibrinogen adsorption amount ($\mu$g/cm$^2$) | 1.7 | 0.8 | 3.8 | 0.8 | 1.7 |
| PP: polypropylene<br>PMEA: poly-2-methoxyethyl acrylate<br>MPC polymer: a copolymer of 2-methacryloyloxyethyl phosphorylcholine and butyl methacrylate | | | | | |

Evaluation of Polyurethane Films Formed Using Aqueous Polyurethane Dispersions

Aqueous dispersions of the polymer compositions obtained in Examples 1 to 17 were individually placed into a Petri dish coated with Teflon (registered trademark) to a film thickness after drying of 200 pm, dried at 20°C for 3 days and at 80°C for 1 hour, and then further dried at 120°C for 30 minutes to allow urethane prepolymer particles dispersed in the aqueous dispersion to fuse to each other, thus forming a polyurethane film. Using the polyurethane film, the following tests were carried out.

Evaluation of Platelet Adhesion Index

A platelet adhesion test was performed by the following method. Whole human blood, blood purchased for experiments collected in the U.S., was used for an experiment within 5 days after the blood collection. The refrigerated human whole blood was allowed to stand at room temperature for about 30 minutes to return the blood temperature to room temperature. The blood was then inverted for mixing 3 times and centrifuged with a centrifugal separator (tabletop centrifuge model 2420, produced by Kubota Corporation) at 1500 rpm for 5 minutes. About 500 $\mu$L of the (light-yellow, translucent) supernatant obtained was then collected and used as a platelet-rich plasma (PRP). After collection, the solution was further centrifuged at 4000 rpm for 10 minutes, and about 2 mL of the (light-yellow, transparent) supernatant was collected and used as a platelet-poor plasma (PPP). The platelet concentration in PRP was calculated by counting platelets in PRP diluted 800 times with PBS (-) using a hemocytometer. The PRP was diluted with PPP to a seeding concentration of $4.0 \times 10^7$ cells/cm$^2$ to prepare a platelet suspension.

Each of the polyurethane films prepared above was peeled off from the Petri dish and sufficiently wetted in advance with physiological saline. 450 $\mu$L (about 300 $\mu$L/cm$^2$) of the platelet suspension prepared above was placed on the surface of each polyurethane film and incubated at 37°C for 1 hour to adhere platelets. Then, after the platelet suspension was removed and washed twice with PBS, the resulting mixture was immersed in 1% glutaraldehyde (25% glutaraldehyde, produced by Polysciences, Inc., 01909 diluted to 1/25 with PBS (-)) solution and incubated at 37°C for 2 hours to immobilize the adhered platelets onto the substrate. After the immobilization, washing was performed by immersing each platelet-coated substrate into PBS (-) (10 minutes), a mixture of PBS (-) and water at a ratio of 1:1 (8 minutes), and water (8 minutes and 10 minutes) one time each. After washing, the platelet-coated substrate was air-dried for 3 hours, and then dried in a vessel containing silica gel for at least 1 day. After drying, the surface of the substrate was observed with a scanning electron microscope (SEM, KEYENCE, 3D real surface view microscope VE-9800) to count the number of platelets adhered to the surface of each polyurethane film.

On the other hand, the number of platelets adhered to a PET (polyethylene terephthalate) film surface, which is known not to show hemocompatibility, when platelets were allowed to adhere thereto in the same manner as above, was counted and used as a negative control. A value standardized by dividing the number of platelets adhered to each polyurethane film surface by the number of platelets counted as being adsorbed on the PET surface was defined as a "platelet adhesion index." According to the platelet adhesion index, a difference in the frequency of platelet adhesion derived from the state of blood used for evaluation is excluded, and the degree of hemocompatibility indicated by the sample surface can be appropriately evaluated.

Further, in order to clarify the degree of platelet adhesion to a surface showing biocompatibility, PMEA, which is known to show high biocompatibility, and a polymer (an MPC polymer) obtained by copolymerizing the MPC with BMA (butyl methacrylate) and making the copolymer insoluble in water were also subjected to a platelet adhesion test at the same time as performing the above test, and these were used as positive controls.

Measurement of Water Resistance

Each of the polyurethane films prepared above was peeled off from the Petri dish, cut into a predetermined size (2 cm × 4 cm), and used as an evaluation sample. The weight of each evaluation sample before and after immersion in tap water (20°C) as a test liquid for 4 hours was measured. The weight increase rate was obtained according to the following formula to evaluate the water resistance of each polyurethane film.

$$\text{Weight increase rate} = (\text{Weight after immersion - Weight before immersion}) / \text{Weight before Immersion} \times 100 \, (\%)$$

Measurement of Mechanical Properties

The polyurethane films prepared above were peeled off from the Petri dish and cut into strips 10 mm in width and 100 mm in length in accordance with JIS K6301 (2010) to prepare evaluation samples for a tensile test. In the test, a tensile test was carried out at a temperature of 23°C (relative humidity: 55%) at a distance between chucks of 50 mm and a tensile speed of 500 mm/min using a tensile tester (product name: TENSILON UTM-III-100, produced by Orientec Co., Ltd.). The maximum tensile stress that each sample exhibited was measured, and the maximum tensile stress was divided by the initial cross-sectional area of the sample to obtain the maximum point intensity ($N/mm^2$).

Tables 6 and 7 shows the results of the above tests.

Table 6

| | Examples | | | | | | | | | |
|---|---|---|---|---|---|---|---|---|---|---|
| | 1 | 2 | 3 | 4 | 5 | 6 | 7 | 8 | 9 | 10 |
| Platelet adhesion | 1.7 | 10.2 | 6.9 | 4.5 | 1.8 | 12.7 | 4.5 | 5.4 | 38.5 | 26.9 |
| Mass increase (%) | 59 | 123 | 26 | 101 | 35 | 9 | 4 | 21 | 8 | 5 |
| Maximum point intensity ($N/mm^2$) | 7.6 | 4.2 | 18.7 | 7.0 | 9.4 | 39.2 | 27.6 | 31.9 | 36.7 | 20.0 |

Table 7

| | Examples | | | | | | | PMEA | MPC polymer | PET |
|---|---|---|---|---|---|---|---|---|---|---|
| | 11 | 12 | 13 | 14 | 15 | 16 | 17 | | | |
| Platelet adhesion | 1.7 | 1.32 | 10.9 | 0.8 | 1.0 | 7.0 | 13.1 | 0.45 to 15.38 | 0.83 to 15.27 | 100 |
| Mass increase (%) | 21 | 96 | 23 | 108 | 87 | 120 | 72 | - | - | - |
| Maximum point intensity ($N/mm^2$) | 17.3 | 1.5 | 29.6 | 0.6 | 2.1 | 0.1 | 0.9 | - | - | - |

Evaluation Results

(1) Platelet Adhesion Test

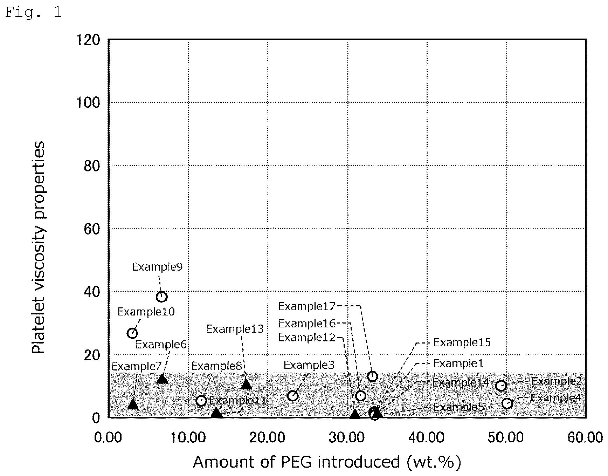

Fig. 1 shows the results of plotting the platelet adhesion index on the surface of each polyurethane film formed by the polymer composition of the present invention relative to the amount of polyoxyethylene structure introduced into the polymer composition. In Figs. 1, 3, and 4, the mass ratio of the PEG used as the PEG component and the branched PEG to the total mass of the polyol containing the PEG component and the polyisocyanate used in producing each polymer is described as the "the amount of PEG introduced (wt%)."

Fig. 1 shows the degree of number of platelet adhesion observed on each sample surface (platelet adhesion index), with the number of platelets adhered to the PET surface being defined as 100. In Fig. 1 and other figures, "o" indicates a polymer having a polyoxyethylene structure in the polymer main chain, and "▲" indicates a polymer having a polyoxyethylene structure in a side chain portion of the polymer.

As shown in Fig. 1, the results of films formed of a urethane resin having a urethane bond in the main chain and having a polyoxyethylene structure in the main chain and/or a side chain thereof show that while a difference in platelet adhesion index occurs in accordance with the difference in the polymer structure and the amount of PEG introduced, all of the films show a platelet adhesion index of 40 or less.

Further, as shown in Table 7, while PMEA and MPC polymers showing high biocompatibility exhibited a platelet adhesion index of about 15 or less (gray range in Fig. 1), the polymer composition having a predetermined formulation according to the present invention is presumed to show high biocompatibility because the polymer composition of the present invention had a platelet adhesion index that was equal to or less than the platelet adhesion index of the PMEA or MPC polymer.

In particular, in the polymer in which a polyoxyethylene structure is introduced into the side chain portion of the polymer (▲), even if the amount of the polyoxyethylene structure introduced is 3.0 wt% (Example 7), the polymer (▲) shows a platelet adhesion index equal to or less than that of PMEA or the like, and it is inferred that the introduction of a small amount of a polyoxyethylene structure provides high biocompatibility.

(2) Strength Test of Polyurethane Film

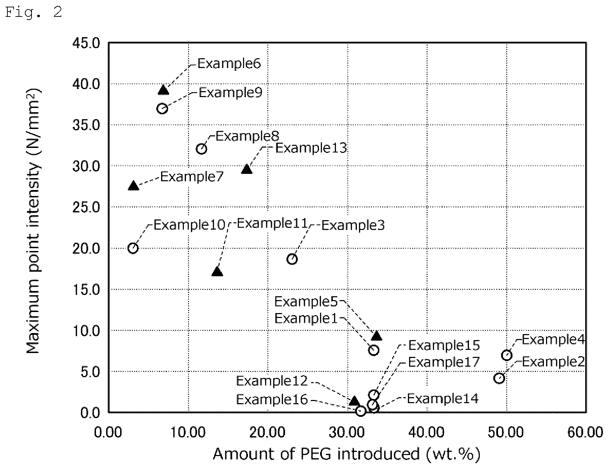

Fig. 2 shows the relationship between the maximum point intensity of each polyurethane film formed of the polymer composition of the present invention and the amount (wt%) of PEG (polyoxyethylene structure) introduced into the polyurethane. As shown in Fig. 2, it was observed that in general, the maximum point intensity tended to decrease with an increase in the amount of the polyoxyethylene structure introduced. On the other hand, the results show that when the amount of PEG introduced is 30 wt% or less, strength equivalent to that of the polyurethane film having no polyoxyethylene structure introduced therein is maintained.

Further, the maximum point intensity tends to be high, in particular, in a polymer in which a polycarbonate polyol is used as a polyol other than the PEG component and an aliphatic polyisocyanate is used as a polyisocyanate, or in a polymer in which a polyoxyethylene structure is introduced into a side chain portion of the polymer.

(3) Water Resistance Evaluation Test

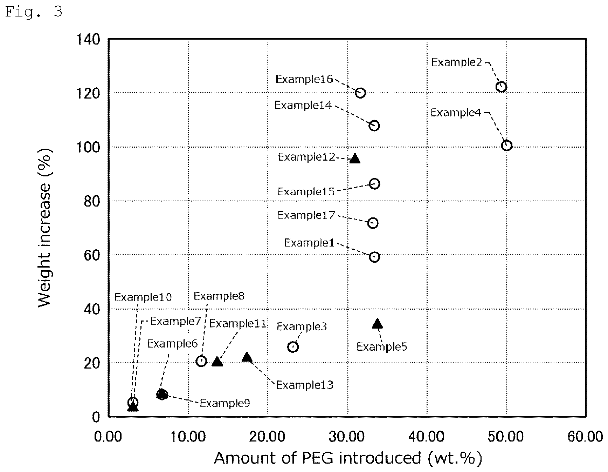

Fig. 3 shows the relationship between the weight increase rate of each polyurethane film formed of the polymer composition of the present invention when the polyurethane film contains water upon immersion in water and the amount (wt%) of PEG introduced into the polyurethane. As shown in Fig. 3, it was observed that in general, as the amount of PEG introduced increases, the water content upon immersion in water increases and the water resistance tends to decrease. On the other hand, the results showed that when the amount of PEG introduced was set to 30 wt% or less, the weight increase rate was suppressed and a predetermined water resistance was provided. Further, none of the polymer compositions were observed to be dissolved in the aqueous phase, and exhibited water insolubility.

Further, the results show that in particular, a polymer prepared using a polycarbonate polyol as a polyol other than the PEG component and using an aliphatic polyisocyanate as a polyisocyanate has a lower weight increase rate due to water content than a polymer using a polyol/polyisocyanate having another structure.

Further, in particular, in a polymer in which a polyoxyethylene structure was introduced into a side chain portion of the polymer (Example 5), an increase in the weight increase rate that would occur with an increase in amount of the polyoxyethylene structure introduced tended to be suppressed.

Preparation and Evaluation of Medical Composition B

Example 18

An aqueous polyurethane dispersion was produced according to the kind and proportion (parts by mass) of each starting material component shown in Table 8. Polycarbonate diol of 1,6-hexanediol (PCD (1,6-HD), produced by Ube Corporation, product name: ETERNACOLL UH-100, number average molecular weight: about 1000, average hydroxyl value: 110 mg KOH/g) as a polyol component (component A) and hexamethylene diisocyanate (HMDI) (Duranate 50 MS, produced by Asahi Kasei Chemicals Corporation) were added at a molar ratio of 45.41:54.59 to a four-necked flask equipped with a stirrer, a reflux condenser, a thermometer, and a nitrogen-feeding tube. Subsequently, methyl ethyl ketone (MEK) was added in such an amount to achieve a solids content of 80 mass%, thus obtaining a uniform starting material liquid. An organotin compound (bis(neodecanoyloxy)dioctylstannane, produced by Songwon Industrial Co., Ltd.) as a reaction catalyst was added to the starting material liquid in an amount of 0.002 mass% based on the total mass of 1,6-HD and HMDI. The temperature was raised to 65°C, and the reaction was started at this temperature. After confirming that the reaction had been carried out at a temperature of 70°C to 77°C until the predetermined free isocyanate group content (%) was achieved, the resulting mixture was cooled to obtain a solution.

This solution was cooled to 45°C. A surfactant (product name: Neugen EA-157, produced by DKS Co. Ltd.) was added to the solution in an amount of 20 mass% based on the total mass of 1,6-HD and HMDI. After the resulting mixture was fully mixed, 400 mass% of water was added as a chain extender and stirring was performed at a high speed with a homomixer for 1 hour to allow a chain extension reaction to proceed, thus obtaining an aqueous dispersion. After a silicone antifoaming agent (silicone emulsion, produced by Dow Corning Toray Co., Ltd.) was added to the obtained aqueous dispersion, MEK was distilled off by heating under reduced pressure with an evaporator to obtain an aqueous polyurethane dispersion.

Table 8

| Starting materials | | | Examples | | | | PMEA | MPC polymer | PET |
|---|---|---|---|---|---|---|---|---|---|
| | | | 18 | 19 | 20 | 21 | | | |
| Component A | PCD (1,6-HD) | Mw = 1000 | 83.2 | 49.59 | 49.59 | 71.39 | | | |
| Component B | HMDI | Mw = 168.19 | 16.8 | 16.68 | 16.68 | 54.59 | | | |
| PEG | Branched PEG | Mw = 1020 | | 33.73 | 33.73 | 11.83 | | | |
| Reaction catalyst | | | 0.002 | 0.00 | 0.00 | 0.00 | | | |
| Surfactant | | | 20 | | | 8.00 | | | |
| Defoaming agent | | | 0.01 | 0.01 | 0.01 | 0.01 | | | |
| Chain extender | | | Water | DETA | Water | Water | | | |
| Urethane resin | Number of terminal functional groups (NCO) | | 2.00 | 2.00 | 2.00 | 2.00 | | | |
| | Molecular weight (Mn) | | 5935 | 6050 | 6050 | 5952 | | | |
| | [NCO]/[OH] | | 1.20 | 1.20 | 1.20 | 1.20 | | | |
| | Number of free NCO groups (%) | | 1.42 | 1.39 | 1.39 | 1.41 | | | |
| Water dispersion analysis value | Appearance | | - | Translucent liquid | Translucent liquid | Milky white liquid | | | |
| | Non-volatile content (%) | | - | 26.5 | 26.5 | | | | |
| | pH (stock solution) | | - | 8.1 | 6.3 | 6.4 | | | |
| | Viscosity (mPa · s / 20°C) | | | 38 | 30 | 70 | | | |
| | Average particle size ($\mu$m) | | - | 0.02 | 0.02 | 0.08 | | | |
| | 90% cumulative average particle size ($\mu$m) | | - | 0.04 | 0.03 | 0.14 | | | |
| | 95% cumulative average particle size ($\mu$m) | | - | 0.05 | 0.03 | 0.17 | | | |

| Starting materials | | | Examples | | | | PMEA | MPC polymer | PET |
|---|---|---|---|---|---|---|---|---|---|
| | | | 18 | 19 | 20 | 21 | | | |
| Physical properties of the film (film thickness: about 500 μm)*3 | Tg (°C)): DSC | | -45 | -49 | -51 | -46 | | | |
| | Water resistance (weight increase) (%) | 4 °C | 10 | 98 | 56 | 17 | | | |
| | | 37 °C | 9 | 101 | 65 | 15 | | | |
| | Tactile sensation of water-containing film (qualitative) | 4 °C | Slightly tucked | Slightly tucked | Slightly tucked | Slightly tucked | | | |
| | | 37 °C | Slightly tucked | Slightly tucked | Slightly tucked | Slightly tucked | | | |
| | Water content (%) | 4 °C | 8.8 | 49.4 | 35.9 | 14.8 | | | |
| | | 37 °C | 8.4 | 50.1 | 39.3 | 12.9 | | | |
| | Young's modulus (Mpa) | | 58 | 1 | 8 | 25 | | | |
| | Maximum point intensity (N/mm$^2$) | | 33.7 | 0.3 | 13.4 | 41.9 | | | |
| | Breaking elongation (%) | | 972 | 804 | 1000 | 1044 | | | |
| | 100% stretch stress (Mpa) | | 5.4 | 0.3 | 1.1 | 3.2 | | | |
| | 200% stretch stress (Mpa) | | 6.8 | 0.2 | 1.4 | 4.4 | | | |
| | 300% stretch stress (Mpa) | | 9.0 | 0.2 | 1.8 | 6.3 | | | |
| Platelet adhesion index | | | 6.50 | 0.12 | 0.48 | 2.05 | 30.08 | 2.53 | 100.00 |

EP 4 316 541 A1

Example 19

An aqueous polyurethane dispersion was prepared according to the formulation shown in Table 8. Using a four-necked flask equipped with a stirrer, a reflux condenser, a thermometer, and a nitrogen-feeding tube, 49.59 parts by mass of polycarbonate diol of 1,6-hexanediol (PCD(1,6-HD), produced by Ube Corporation, product name: ETERNACOLL UH-100, number average molecular weight: about 1000, average hydroxyl value: 110 mg KOH/g) as a polyol component (component A), 33.73 parts by mass of polyethylene glycol (produced by DKS Co. Ltd., product name: PEG-1000, number average molecular weight: about 1000, average hydroxyl value: 110 mg KOH/g)), 16.68 parts by mass of hexamethylene diisocyanate (HMDI) (Duranate 50 MS, produced by Asahi Kasei Chemicals Corporation) as a polyisocyanate component (B), and 0.003 parts by mass of an organotin compound (bis(neodecanoyloxy)dioctylstannane, produced by Songwon Industrial Co., Ltd.) as a reaction catalyst were added to 90 parts by mass of methyl ethyl ketone as a reaction medium. A reaction was allowed to proceed at 75°C for 4 hours to produce a urethane prepolymer, thus obtaining a starting material liquid obtained by dissolving the urethane prepolymer in methyl ethyl ketone. The branched PEG is a diol (Ymer N120, produced by Perstorp Co., Ltd.) having a structure in which hydrogen atoms of an alkylene chain having hydroxyl groups at both ends are replaced by, for example, a group containing a polyoxyethylene structure, and PEG having a number average molecular weight of about 1020 and an average hydroxyl value of 110 mg KOH/g was used.

The starting material liquid was cooled to 45°C. While 300 parts by mass of water and 1.02 parts by mass of diethylenetriamine (DETA) were gradually added to a mixture of the starting material liquid and 0.01 parts by mass of a silicone antifoaming agent (silicone emulsion, produced by Dow Corning Toray Co., Ltd.), a urethane prepolymer was emulsified and dispersed in a liquid phase using a homogenizer to obtain an aqueous dispersion. Subsequently, after a chain extension reaction with DETA was carried out for 1 hour, the resulting reaction mixture was maintained in an environment of 50°C under reduced pressure to remove methyl ethyl ketone as a reaction medium by evaporation, thus obtaining an aqueous polyurethane dispersion containing about 26.5 mass% of a non-volatile component.

Examples 20 to 21

Aqueous polyurethane dispersions were prepared in the same manner as in Example 19 except that the proportions of PCD (1,6-HD) and HMDI, the proportion of branch PEG to be used, and the kind, the proportion, the average molecular weight, the structure, etc., of the chain extender to be used were varied. Table 8 shows the details of the starting material components used in Examples 19 to 21 (parts by weight).

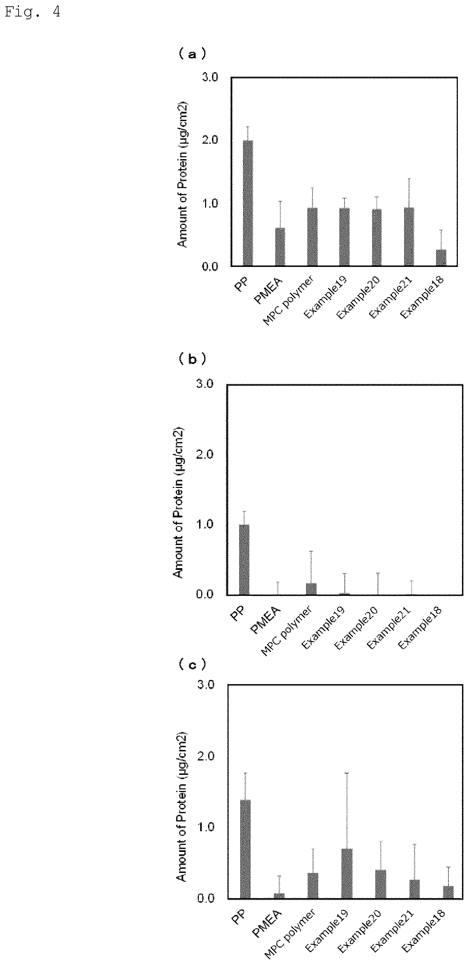

Fig. 4 shows the results of protein adsorption tests; (a) shows the results of an albumin adsorption test, (b) shows the results of a fibrinogen adsorption test, and (c) shows the results of a fibronectin adsorption test. The aqueous polyurethane dispersion obtained in Example 18 was dried to separate a urethane resin, and the urethane resin was then dissolved in methylene chloride to prepare a 7 mass% urethane resin dispersion of methylene chloride dispersion. A polyurethane film was prepared using the methylene chloride dispersion. For reference, Fig. 4 also shows test results of protein adsorption on the PMEA, MPC polymer, and PET.

Table 8 shows various analytical values of the aqueous polyurethane dispersions obtained in Examples 18 to 21, various physical properties of the urethane resin films formed of the dispersions, and measurement results of the platelet adhesion index. For reference, Table 8 also shows results of the platelet adhesion index of the PMEA, MPC polymer, and PET.

The results of Table 8 and Fig. 4 show that a film comprising a urethane resin having a structural unit derived from a polycarbonate polyol and a structural unit derived from an aliphatic isocyanate and having at least one urea bond has excellent mechanical strength and is inhibited from platelet adsorption and adsorption of various proteins. The results clarify that the film has excellent mechanical strength and biocompatibility.

Comparative Example 1

A mixture composed of polycarbonate diol of 1,6-hexanediol (PCD(1,6-HD), produced by Ube Corporation, product name: ETERNACOLL UH-100, number average molecular weight: about 1000, average hydroxyl value: 110 mg KOH/g) and tolylene diisocyanate (TDI) at a molar ratio of 45.46:54.54 was placed into a four-necked flask equipped with a stirrer, a reflux condenser, a thermometer, and a nitrogen-feeding tube. Subsequently, methyl ethyl ketone (MEK) was added to the flask to achieve a solids content of 80 mass%, thus obtaining a uniform starting material liquid. An organotin compound (bis(neodecanoyloxy)dioctylstannane, produced by Songwon Industrial Co., Ltd.) was added as a reaction catalyst to the starting material liquid to achieve a concentration of 0.002 mass% based on the total mass of the mixture, and the temperature was raised to 65°C. The reaction was started at this temperature. After confirming that the reaction had been carried out at a temperature between 70°C and 77°C until the predetermined free isocyanate group content (%) was achieved, the resulting mixture was cooled to obtain a solution.

This solution was cooled to 45°C. A surfactant (product name: Neugen EA-157, produced by DKS Co. Ltd.) was added to the solution in an amount of 20 mass% based on the total mass of the mixture. After the resulting mixture was thoroughly mixed, 400 mass% of water as a chain extender was added. The resulting mixture was stirred at a high speed with a homomixer for 1 hour to allow a chain extension reaction to proceed, thus obtaining an aqueous dispersion. After a silicone antifoaming agent (silicone emulsion, produced by Dow Corning Toray Co., Ltd.) was added to the obtained aqueous dispersion, MEK was distilled off by heating under reduced pressure with an evaporator to obtain an aqueous polyurethane dispersion.

Comparative Example 2

An aqueous polyurethane dispersion was obtained in the same manner as in Comparative Example 1 except that a mixture composed of a polyester polyol (MPD/AA; 3-methyl-1,5-pentanediol/adipic acid) and isophorone diisocyanate (IPDI) (molar ratio 45.45:54.55) was used in place of the mixture used in Comparative Example 1, and the amount of the organotin compound used was changed to 0.004 mass% based on the total mass of the mixture.

Comparative Example 3

An aqueous polyurethane dispersion was obtained in the same manner as in Comparative Example 1 except that a mixture composed of polyester polyol (MPD/AA; 3-methyl-1,5-pentanediol/adipic acid) and tolylene diisocyanate (TDI) (molar ratio: 45.44:54.56) was used in place of the mixture used in Comparative Example 1, and the amount of the organotin compound used was changed to 0.0035 mass% based on the total mass of the mixture.

Comparative Example 4

An aqueous polyurethane dispersion was obtained in the same manner as in Comparative Example 1, except that a mixture composed of polytetramethylene glycol (PTMG) and tolylene diisocyanate (TDI) (molar ratio: 45.44:54.56) was used in place of the mixture used in Comparative Example 1, and the amount of the organotin compound used was changed to 0.006 mass%, based on the total mass of the mixture.

Evaluation of Aqueous Polyurethane Dispersions Obtained in Comparative Examples

Using the aqueous polyurethane dispersions obtained in Comparative Examples 1 to 4, polyurethane films were prepared in the same manner as above. However, all of the obtained films were in the form of starch syrup, and the desired polyurethane film could not be obtained. Accordingly, the aqueous polyurethane dispersions obtained in Comparative Examples 1 to 4 were unsuitable for use as medical coating agents.

**Claims**

1. A medical composition comprising a urethane resin, the urethane resin having a urethane bond in a main chain and a polyoxyethylene structure in the main chain and/or a side chain.

2. The medical composition according to claim 1, wherein the urethane resin has a structural unit derived from a polycarbonate polyol.

3. The medical composition according to claim 1, wherein the urethane resin has a structural unit derived from an isocyanate compound.

4. A medical composition comprising a urethane resin, the urethane resin having a structural unit derived from a polycarbonate polyol and a structural unit derived from an aliphatic isocyanate, and having at least one urea bond.

5. The medical composition according to claim 4, wherein the urethane resin has a polyoxyethylene structure in a main chain and/or a side chain.

6. The medical composition according to any one of claims 1 to 5, which is an aqueous dispersion.

7. A protein adsorption inhibitor comprising the medical composition of any one of claims 1 to 6.

8.  A platelet adsorption inhibitor comprising the medical composition of any one of claims 1 to 6.

9.  A medical fiber treatment agent comprising the medical composition of any one of claims 1 to 6.

10. A medical instrument comprising a film of the medical composition of any one of claims 1 to 6.

11. A medical fiber comprising the medical fiber treatment agent of claim 9.

12. A method for inhibiting protein adsorption, comprising forming a film of the medical composition of any one of claims 1 to 6 on a substrate.

13. A method for inhibiting platelet adsorption, comprising forming a film of the medical composition of any one of claims 1 to 6 on a substrate.

14. A method for treating a medical fiber, comprising treating a medical fiber with the medical composition of any one of claims 1 to 6.

Fig. 1

Fig. 2

Fig. 3

Fig. 4

(a)

(b)

(c)

## INTERNATIONAL SEARCH REPORT

International application No.

**PCT/JP2022/016184**

### A. CLASSIFICATION OF SUBJECT MATTER

*A61L 33/06*(2006.01)i; *A61L 31/10*(2006.01)i; *A61L 33/14*(2006.01)i; *C08G 18/42*(2006.01)n; *C08G 18/48*(2006.01)n
FI: A61L33/06 300; A61L31/10; A61L33/14; C08G18/42; C08G18/48 033

According to International Patent Classification (IPC) or to both national classification and IPC

### B. FIELDS SEARCHED

Minimum documentation searched (classification system followed by classification symbols)

A61L33/06; A61L31/10; A61L33/14; C08G18/42; C08G18/48

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/MEDLINE/EMBASE/BIOSIS (STN), JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | VAKILI, H. et al. Enhanced hemocompatibility of a PEGilated polycarbonate based segmented polyurethane. International Journal of Polymeric Materials and Polymeric Biomaterials. 2020, 71:7, 531-539<br>    abstract, table 1, fig. 8, p. 532, right column, "2.1. Synthesis" | 1-14 |
| Y | | 7, 12 |
| X | XU, Y. et al. Synthesis of polycarbonate urethanes with functional poly(ethylene glycol) side chains intended for bioconjugates. Polymer. 2013, 54, 5363-5373<br>    fig. 1, 8, p. 5372, left column, lines 33-35 | 1-3, 6, 8-11, 13-14 |
| Y | | 4-5, 7, 12 |
| Y | JP 7-53764 A (ASAHI CHEM. IND. CO., LTD.) 28 February 1995 (1995-02-28)<br>    paragraphs [0006]-[0008] | 4-5 |

☐ Further documents are listed in the continuation of Box C.          ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **11 May 2022** | **24 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/016184**

| Patent document cited in search report | Publication date (day/month/year) | Patent family member(s) | Publication date (day/month/year) |
|---|---|---|---|
| JP 7-53764 A | 28 February 1995 | (Family: none) | |

Form PCT/ISA/210 (patent family annex) (January 2015)

30

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Journal of the Adhesion Society of Japan,* 2015, vol. 51 (9), 15-25